(19) Europäisches Patentamt
European Patent Office
Office européen des brevets

(11) Numéro de publication: **0 089 894**
**B1**

(12) # FASCICULE DE BREVET EUROPEEN

(45) Date de publication du fascicule du brevet: **11.06.86**

(51) Int. Cl.⁴: **C 07 D 235/06**, A 61 K 31/415

(21) Numéro de dépôt: **83400578.7**

(22) Date de dépôt: **21.03.83**

(54) **(Benzimidazolyl-1)-1,N-((hydroxy-4 méthoxy-3 phényl)-2 hydroxy-2 éthyl) amino-3 butane et ses sels et hydrates à activité bêta-adrénergique, leurs applications thérapeutiques, et procédé pour les préparer.**

(30) Priorité: **24.03.82 FR 8205035**

(43) Date de publication de la demande: **28.09.83 Bulletin 83/39**

(45) Mention de la délivrance du brevet: **11.06.86 Bulletin 86/24**

(84) Etats contractants désignés: **AT BE CH DE GB IT LI LU NL SE**

(56) Documents cités:
**EP - A - 0 008 653**
**EP - A - 0 034 116**
**DE - A - 2 238 504**

**Le dossier contient des informations techniques présentées postérieurement au dépôt de la demande et ne figurant pas dans le présent fascicule.**

(73) Titulaire: **Société anonyme dite: LABORATOIRE ROGER BELLON, 159, avenue du Roule B.P. 105, F-92201 Neuilly-sur-Seine (FR)**

(72) Inventeur: **Joannic, Michel, 88, rue du 11 Novembre, F-93330 Neuilly-sur-Marne (FR)**
Inventeur: **Roquet, Françoise, 142, Boulevard Masséna, F-75643 Paris Cedex 13 (FR)**
Inventeur: **Pesson, Marcel, 5, rue Armand Carrel, F-75019 Paris (FR)**

(74) Mandataire: **Doan, Dinh Nam et al, Cabinet BEAU DE LOMENIE 55, rue d'Amsterdam, F-75008 Paris (FR)**

**EP 0 089 894 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention a pour objet le [benzimidazolyl-1]-1, N-[hydroxy-4 méthoxy-3 phényl)-2 hydroxy-2 éthyl]amino-3 butane de formule (I), ses sels d'addition d'acides minéraux ou organiques, non toxiques, pharmaceutiquement acceptables et les hydrates desdits sels, présentant des propriétésβ-adrénergiques remarquables et susceptibles d'applications thérapeutiques :

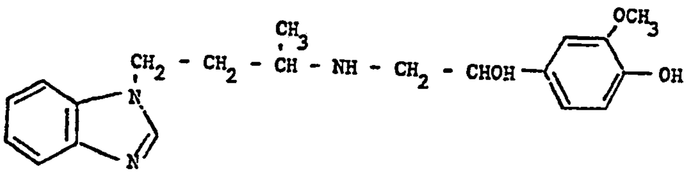

(I)

Le composé (I) étant dibasique, donne avec les acides, plusieurs types de sels, dont les caractéristiques physicochimiques peuvent rendre l'utilisation thérapeutique plus ou moins aisée.

D'une façon générale, on peut définir un acide par la formule $AH_n$, où A représente un radical minéral ou organique et H un proton acide, n' est un nombre entier (dans le cas des monoacides, n' = 1 ; dans le cas des diacides, n' = 2).

Selon la nature des acides et le degré de salification, les sels du composé (I) répondent à la formule générale (II), où n et n'' représentent des nombres entiers :

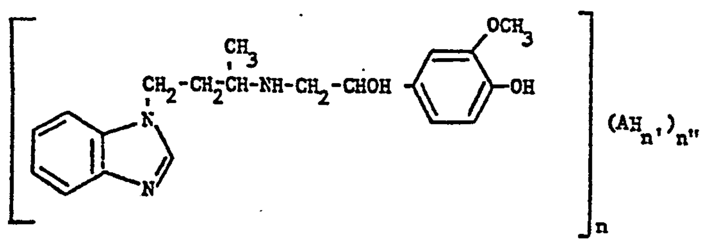

(II

On est ainsi amené à distinguer :

*Les sels "neutres"* dans lesquels, pour les monoacides (n'=1), n=1 et n'=2 ; dans le cas des diacides (n'=2), n=1 et n''=1.

*Les sels "basiques"*, dans lesquels, pour les monoacides (n'=1), n=1 et n''=1 ; dans les cas des diacides (n'=2), n=2, n''=1.

Ces sels, quel que soit leur type, peuvent former des hydrates. Ils peuvent également être solvatés par les alcools inférieurs (tels que le méthanol ou l'éthanol), qui souvent employés pour leur préparation ou leur purification.

Comme sels d'addition d'acide non toxiques du produit de formule I selon l'invention, on peut citer notamment les sels avec les acides minéraux suivants : acide chlorhydrique, acide bromhydrique, acide sulfurique, acide phosphorique, et les sels avec les acides organiques suivants : acide maléïque, acide fumarique, acide tartrique, acide citrique, acide oxalique, acide succinique, acide mandélique acide méthane sulfonique, acide benzène sulfonique, acide p.toluène sulfonique, acide camphosulfonique, etc....

Certains sels pouvant être solvatés par des solvants de recristallisatic il est donc préférable de choisir un acide tel que le sel correspondant ne soit pas solvaté.

Certains composés (benzimidazolyl-1) alkylamino éthanols substitués utiles comme bronchospasmolytiques et relaxants musculaires sont décrits dans le brevet européen N° 8653.

Le composé selon l'invention est caractérisé par la présence d'un atome de carbone asymétrique en position 3 de la portion amino butane et des substituants hydroxy-4 et méthoxy-3 sur la portion phényle.

Le composé selon l'invention et ses sels, où A représente un radical non susceptible d'être réduit par hydrogénation catalytique, sont préparés selon les étapes suivantes :

a) l'amino-3 [benzimidazolyl-1]-1 butane (III) (1 mole) est condensé, selon un procédé connu en soi, en milieu alcoolique, avec le (méthoxy-3 phénylméthoxy-4) phénylglyoxal ($IV_a$) (1-1,1 mole), ou son acétal éthylique ($IV_b$) (éthoxy-2 hydroxy-2 (méthoxy-3 phénylméthoxy-4 phényl)-1 éthanone) (1-1,1 mole). Cette réaction conduit à une imine (V), qui n'est pas isolée, et est réduite "in situ" selon un procédé connu, par un excès (2 à 4 MOLES) de borohydrure de sodium.

Cette réduction porte à la fois sur la fonction imine et sur le groupe carbonyle cétonique. On obtient ainsi le [benzimidaxolyl-1]-1 N-[hydroxy-2 (méthoxy-3 phénylméthoxy-4 phényl)-2 éthyl] amino-3 butane (VI)

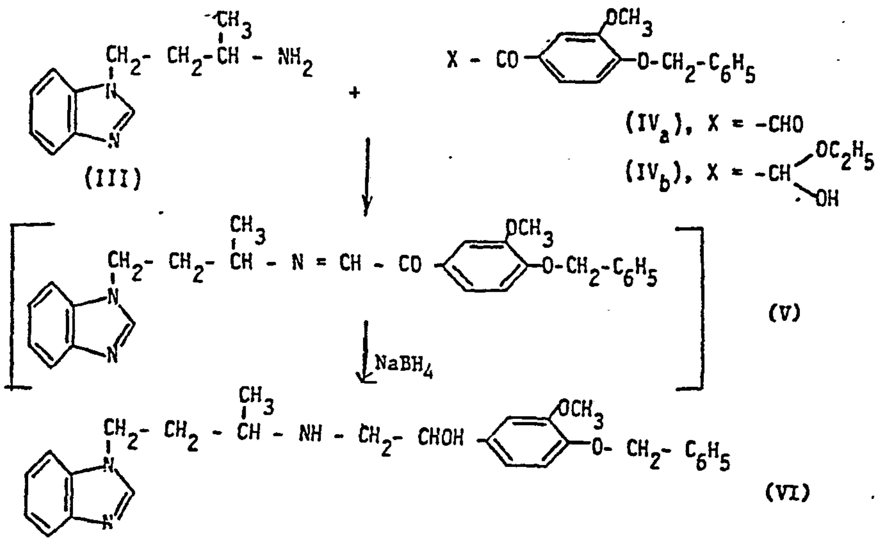


(III) (IVa), X = -CHO (IVb) (V) (VI)

b) La base (VI), huileuse, est transformée en un sel cristallisé (VII). c) L'hydrogénolyse de ce dernier fournit le sel correspondant du produit selon l'invention. Cette opération est conduite, de préférence, en solution ou suspension hydroalcoolique (contenant de 20 à 50% d'eau), en présence de charbon palladié à 5 ou 10% (utilisé à raison de 5 à 10% du poids du produit mis en oeuvre). On opère le plus souvent sous des pressions d'hydrogène comprises entre 1 et 50 bars et des températures situées entre 20 et 40°C.

Quand l'hydrogénation est terminée, la solution est filtrée pour séparer le catalyseur, puis elle est concentrée à sec sous vide. Le résidu, généralement visqueux, est repris par un solvant convenable, pour amener la cristallisation du sel (II), qui est purifié par recristallisation.

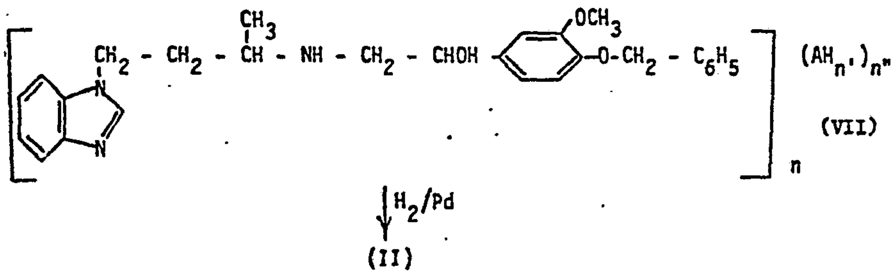


(VII) (II)

Les sels (II), dont le reste acide A est susceptible d'être réduit par hydrogénation catalytique, ne peuvent pas être obtenus par la méthode décrite ci-dessus. Ils sont préparés, selon un procédé connu, par salification de la base (I). Cette dernière est elle-même obtenue, par alcalinisation de la solution aqueuse d'un des sels décrits ci-dessus, au moyen d'un carbonate alcalin ou d'une base faible telle que l'ammoniaque.

Les matières premières nécessaires à la préparation du composé selon l'invention sont obtenues ainsi qu'il est décrit ci-dessous :

1/ *Amino-3 [benzimidazolyl-1]-1 butane* (III).

Le benzimidazole (VIII) (1 mole), condensé avec la méthylvinylcétone en léger excès (1-1,1 mole), en présence d'une quantité catalytique de Triton B (hydroxyde de benzyltriméthylammonium), donne le [benzimidazolyl-1]-1 oxo-3 butane (IX). Cette réaction est conduite au sein d'un solvant neutre apolaire, tel qu'un hydrocarbure aromatique (par exemple le toluène) ou, de préférence, l'acétate d'thyle. La cétone (IX) est transformée, selon un procédé connu, en son oxime (X). Cette dernière, par hydrogénation catalytique, en milieu alcoolique, en présence d'ammoniaque et de nickel de Raney, sous pression (20–80 bars) et à chaud (50 à 100°C), conduit à l'amine (III).

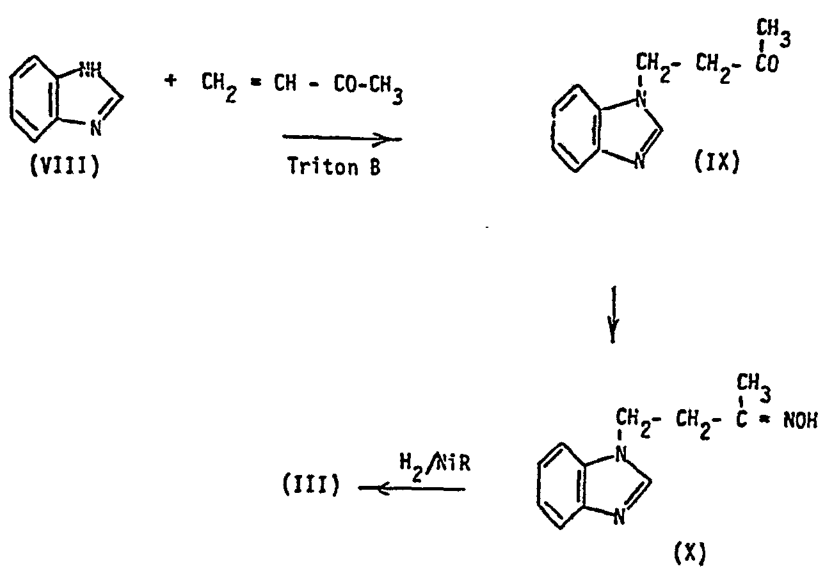


(VIII) (IX) (III) (X)

2/ *(Méthoxy-3 phénylméthoxy-4) phénylglyoxal (IVa) et son acétal éthylique-[éthoxy-2 hydroxy-2 (méthoxy-3 phénylméthoxy-4 phényl)-1 éthanone]* ($IV_b$)

Ces composés nouveaux sont préparés, selon des procédés connus, à partir de la (méthoxy-3 phénylméthoxy-4 phényl)-1 éthanone (XI) :

a) par oxydation, au moyen de l'oxyde de sélénium, à 100°C, dans un solvant convenable, de préférence le dioxanne, la cétone (XI) conduit directement au glyoxal ($IV_a$), qui, à l'air, donne une hémihydrate.

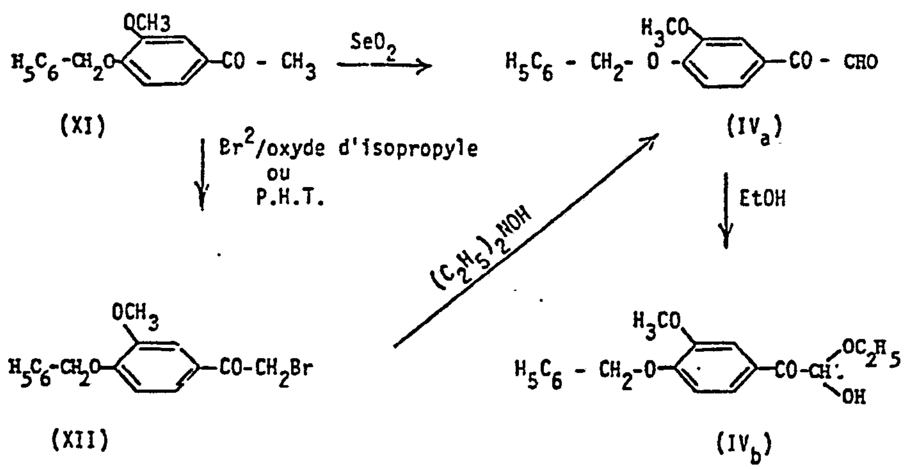


b) La cétone (XI), traitée par le brome, en présence d'un oxyde d'alkyle inférieur, ou mieux, par le pyrrolidone-2 hydrotribromure (P.H.T.) dans un hydrocarbure gras chloré (par exemple le chloroforme), fournit la bromocétone (XII). Cette dernière, par action de la NN-diéthylhydroxylamine, selon un procédé connu [V.E. GUNN et J.P. ANSELME J. Org. Chem. 1977, *42*, 754–755] donne le glyoxal ($IV_a$), qui, traité par l'éthanol, fournit l'hémiacétal ($IV_b$) plus facilement purifiable que le glyoxal lui-même ou son hémihydrate.

Le composé selon l'invention et ses sels présentent les propriétés caractéristiques des substances β-mimétiques *"in vitro"* et *"in vivo"* :

– Relaxation de la trachée et de l'utérus isolés, action chronotrope positive sur l'oreillette isolée.

– Antagonisme du bronchospasme histaminique, action utérorelaxante et tachycardie chez l'animal éveillé ou anesthésié.

– Cette spécificité β mimétique est prouvée par l'effet antagoniste des agents β bloquants.

– Les actions pharmacologiques des produits selon l'invention, comparées à celles d'une substance type (le Salbutamol), sont caractérisées par des effets cardiostimulants plus faibles. De ce fait, les composés, selon l'invention, ont une sélectivité remarquable vis-à-vis de récepteurs de type $\beta_2$.

En raison de leurs propriétés pharmacologiques, le produit selon l'invention et ses sels peuvent être préconisés comme agents thérapeutiques en pneumologie et en obstétrique, notamment dans le traitement de l'asthme et dans les cas de risques d'accouchement prématuré. Selon les indications, ils pourront être administrés par voie orale, par voie parentérale ou par voie rectale. Pour le traitement des affections respiratoires, ils pourront également être utilisés sous forme d'aérosols.

Pour les traitements par voie orale, l'administration sera fonction du poids du malade et de la gravité du syndrome à traiter. La posologie journalière totale sera comprise entre 1,5 et 30 mg, fragmentée en 2 ou 3 prises.

Pour cette administration orale, le produit actif pourra être présenté sous l'une des formes généralement utilisées à cet effet : comprimés, gélules, dragées, et sirops. Dans ces formes, le principe actif pourra être seul ou associé à divers supports ou véhicules inertes, pharmaceutiquement acceptables. On pourra également y ajouter diverses substances édulcorantes ou parfyms généralement employés à cette fin.

On peut notamment utiliser des comprimés titrant de 0,5 à 10 mg de principe actif et comportant divers excipients tels que : citrate de sodium ou phosphate dicalcique, en même temps que divers désintégrants tels que

: amidon (notamment de tapioca ou de pomme de terre), acide alginique, et certains silicates complexes, ainsi que des agents liants, comme le saccharose, la gomme arabique ou la polyvinylpyrrolidone. Les comprimés pourront également comporter des agents lubrifiants généralement utilisés pour faciliter le pastillage, tels que talc, stéarate de magnésium ou laurylsulfate de sodium.

Des compositions solides semblables peuvent être employées pour emplir des capsules en gélatine dure ou molle.

Dans les solutions aqueuses destinées à l'administration orale, le principe actif sera à une concentration comprise entre 0,1 et 2 mg/cm³, ce qui, pour une mesure de 5 cm³ représente une prise de 0,5 à 10 mg de produit.

Dans ces solutions, le principe actif peut être associé à diverses agents édulcorants, aromatisants ou colorants et, éventuellement, des agents émulsifiants ou de mise en suspension, joints à des diluants tels que : eau, éthanol, propylèneglycol, ou ces substances en associations variées.

Les aérosols seront préparés à partir de solutions aqueuses du principe actif à une concentration telle que, à l'emploi, chaque inhalation corresponde à l'administration de 25 à 500 microgrammes de produit.

Pour le traitement par voie parentérale, le principe actif pourra être administré soit en perfusion (0,5 à 10 mg par 24 heures), soit en injections intraveineuses (0,4 à 8 mg par 24 heures). A cet effet, il sera utlisé en solutions aqueuses, à des concentrations comprises entre /0,02 et 0,4 mg/cm³. Ces solutions pourront être favorisées par l'addition d'adjuvants de solubilisation, tels le N,N diméthylformamide, le propylèneglycol ou le lactamide. Elles seront rendues isotoniques, par addition d'une proportion convenable de chlorure de sodium ou de glucose.

Les préparations finales seront passées sur un filtre à bactéries approprié, tel qu'un filtre en verre fritté ou un filtre garni de terre d'infusoires ou un filtre en porcelaine non vernissée. Parmi les filtres préconisés de préférence, on peut citer les filtres Berkefeld, Chemberland, et le filtre de Seitz, où le fluide est aspiré au travers de la paroi filtrante pour tomber dans un récipient stérile.

Pour l'administration rectale, on utlisera des suppositoires contenant de 0,25 à 5 mg de composé actif par unité. Les excipients pourront être : des huiles naturelles ou durcies, des cires, des graisses, des glycérides semisynthétiques.

Les exemples non limitatifs qui suivent sont donnés pour illustrer la présente invention.

**Exemple 1**

*Sulfate basique de [benzimidazolyl-1]-1,N-[(hydroxy-4 méthoxy-3 phényl)-2 hydroxy-2 éthyl]amino-3 butane, dihydrate* [II, A=$SO_4$, n=2, n'=2, n''=1]

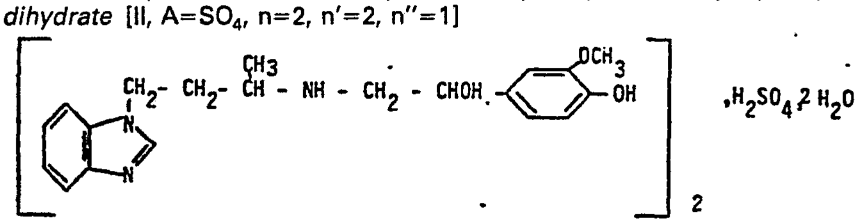

1/ *Sulfate basique de [benzimidazolyl-1]1,N-[méthoxy-3 phénylméthoxy-4 phényl)-2 hydroxy-2 éthyl] amino-3 butane* (VIII, A=$SO_4$, n=2, n'=2, n''=1)

Dans un ballon de 1 litre, muni d'un agitateur, 32,2 g (0,17 mole) d'amino-3 [benzimidazolyl-1]-1 butane sont dissous dans 400 cm³ d'éthanol, on y ajoute 54 g (0,17 mole) d'éthoxy-2 hydroxy-2 (méthoxy-3 phénylméthoxy-4 phényl)-1 éthanone. Le mélange est agité durant 3 h à température ordinaire ; à la fin de la première heure, l'hémiacétal passe complètement en solution.

Le mélange est refroidi à 10°C et on y ajoute, par petites fractions, sous bonne agitation, 12,9 (0,34 mole) de borohydrure de sodium finement pulvérisé. La solution est agitée encore 3 h, puis abandonnée pendant une nuit à la température ordinaire.

La solution est rendue acide par addition de 45 cm³ d'acide méthanesulfonique, puis diluée par 250 cm³ d'eau, pour dissoudre les sels minéraux qui ont précipité. Le mélange est agité 1 h à température ordinaire.

La majeure partie de l'éthanol est éliminée, par concentration sous vide de la solution à environ 1/3 de son volume. Le résidu est additionné de 400 cm³ d'eau. La solution est extraite à l'acétate d'éthyle (6×100 cm³), afin d'éliminer un léger insoluble.

La phase aqueuse est alcalinisée par addition de lessive de soude. La base qui précipite est extraite par l'acétate d'éthyle (200 cm³, puis 3×100 cm³). Les extraits, réunis, sont lavés à l'eau (2×100 cm³), séchés ($MgSO_4$), filtrés et évaporés à sec sous vide, à 100°C.

On obtient 74 g d'une huile visqueuse qui est dissoute dans 180 cm³ d'ethanol. La solution est agitée 1 h avec 35 g de Ni Raney. Cette opération apour but d'éliminer des poisons éventuels du catalyseur utilisé dans l'opération suivante.

La solution est filtrée et le Nickel de Raney lavé par de l'éthanol (2×25 cm³). Les filtrats, réunis, sont concentrés sous vide, jusqu'à un volume de 100 à 150 cm³, puis ramenés à un volume, exactement mesuré, (250 cm³), par addition de la quantité nécessaire d'éthanol. On obtient ainsi la solution (A).

Sur 0,5 cm³ de cette solution, on procède au dosage de la basicité en milieu anhydre (acide acétique), par l'acide perchlorique N/10 (dans l'exemple décrit, il a fallu 5,8 cm³ de $C10_4H$ 0,102 N). A partir du résultat ainsi

obtenu, on calcule la quantité totale d'acide sulfurique nécessaire pour la salification de la moitié des fonctions basiques du [benzimidazolyl-1]-1,N- [(méthoxy-3 phénylméthoxy-4 phényl)-2 hydroxy-2 éthyl] amino-3 butane présent dans la solution (A). Dans l'exemple cité, ce calcul conduit à 7,3 g de $H_2SO_4$.

Ce poids d'acide sulfurique est ajouté, sous refroidissement, au tiers de son poids en eau (5 cm³), puis le mélange est additionné du même volume (5 cm³) d'éthanol froid.

La solution ainsi obtenue est ajoutée, lentement, sous agitation, à la liqueur mère (A). On observe, transitoirement, la formation d'un précipité qui repasse en solution. Cette dernière est chauffée à reflux jusqu'à début d'apparition de cristaux, puis abandonnée pendant 24 h à température ordinaire.

Le précipité est essoré et remis en suspension dans 570 cm³ d'acétone. Le mélange est agité et chauffé à reflux pendant 1 h. Le solide est essoré, lavé à l'acétone et séché. On obtient 54,9 g de produit brut, suffisamment pur pour l'opération suivante, F~ 178–180°C (pâteux).

Pour l'analyse, 1 g de ce sel est recristallisé dans 10 cm³ de méthylcellosolve, F~ 184–185°C (fusion pâteuse).

<u>Analyse</u> pour $C_{54}H_{64}N_6O_{10}S$ (P.M. 989,16)

| | | | | |
|---|---|---|---|---|
| Calculé % | C 65,56 | H 6,52 | N 8,50 | S 3,24 |
| Trouvé % | C 65,43 | H 6,75 | N 8,75 | S 3,38 |

<u>Spectre de R.M.N.</u>* (D.M.S.O.$d_6$) $\delta$ = 8,15 ppm, 1H (N=CH–N) ; $\delta$ 6,5 à 8 ppm, m, 13 H (aromatiques) ; $\delta$ = 4,95 ppm, s, 2H (-O-$\underline{CH_2}$-) ; $\delta$ = 4,65 ppm , m, 1H ( -$\underline{CH}$(OH) - ) ; $\delta$ = 4,25 ppm, t, 2H (—N–$\underline{CH_2}$-), J = 7 Hz ; $\delta$ = 3,7 ppm, s, 3H(-O$\underline{CH_3}$); $\delta$ = 2,8 ppm, m, 3H(-$\underline{CH}$ -NH - $\underline{CH_2}$-); $\delta$ = 2,0 ppm, m, 2H(-$CH_2$- $\underline{CH_2}$ - CH -) ; $\delta$ = 1,1 ppm, d, 3H (-$\underline{CH_3}$), J = 6,5 Hz.

* Les spectres de R.M.N. ont été obtenus sur appareil BRUKER 80 MHz.

2/ *Hydrogénation.* 49,5 g (0,05 mole) du sulfate décrit ci-dessus sont mis en suspension dans 730 cm³ d'éthanol à 50% et agités, en atmosphère d'hydrogène, à la pression ordinaire, à 40°C, en présence de 5 g de charbon palladié à 10%. L'absorption est terminée en 3 h. Après retour à température ordinaire, le catalyseur est séparé par filtration et lavé à l'éthanol (2×50 cm³). La solution est concentrée à sec au Rotavapor.

Le résidu visqueux est repris, sous agitation, par 400 cm³ d'éthanol à 90%. Après dissolution, le sel commence à précipiter ; on laisse la cristallisation s'achever à la température ambiante pendant une nuit.

Le solide (33,1 g) est essoré et séché dans le vide phosphorique. Il est recristallisé, en atmosphère d'azote, dans 140 cm³ d'eau. Il est essoré et séché dans le vide phosphorique, puis remis à l'air (prise d'humidité), jusqu'à poids constant. On obtient 20,6 g de dihydrate, F 150°C.

<u>Analyse</u> pour $C_{40}H_{50}N_6O_6$, $H_2SO_4$, $2H_2O$ (P.M. 844,96)

| | | | | |
|---|---|---|---|---|
| Calculé % | C 56,85 | H 6,68 | N 9,95 | S 3,79 |
| Trouvé % | C 56,67 | H 6,74 | N 10,20 | S 3,76 |

<u>Spectre de R.M.N.</u>($D_2O$) : $\delta$= 8,25 ppm, 1H ( N=CH–N-), 7,87 à 7,25 ppm, m, 4H (aromatiques) ; $\delta$ = 7,1 à 6,75 ppm, m, 3H (aromatiques) ; $\delta$ = 4,80 ppm, m, (-$\underline{CH}$($NH_2$)- + -CH(OH)- + $H_2O$) ; $\delta$= 4,37 ppm, m, 2H (-N–$\underline{CH_2}$-) ; $\delta$ = 3,88 ppm, s,3 H (-$OCH_3$) ; $\delta$ = 3,15 ppm, d, 2H(NH-$\underline{CH_2}$-CHOH), J = 6 Hz ; $\delta$ = 2,25 ppm, m, 2H (N–$CH_2$-$\underline{CH_2}$-) ; $\delta$ = 1,50 ppm, d, 3H (-CH-$\underline{CH_3}$), J = 7 Hz.

Les matières premières nécessaires à la préparation des composés de l'exemplè 1 sont obtenues ainsi qu'il est décrit ci-dessous :

a) *Amino-3 [benzimidazolyl-1]-1 butane*

Dans un ballon de 2 litres, à 3 tubulures, muni d'un agitateur, d'un réfrigérant à reflux, d'une ampoule à brome et d'un thermomètre, on agite une suspension de 118 g (1 mole) de benzimidazole dans 450 cm³ d'acétate d'éthyle. On y ajoute 2 cm³ d'une solution de triton B à 40% dans le méthanol, puis 79 g 92 cm³=1,1 mole) de méthylvinylcétone fraîchement distillée.

Le mélange est amené à 40°C, température à laquelle la réaction se déclenche avec échauffement. On arrête le chauffage, la température s'élève à 70°C ; lorsqu'elle est revenue à 40°C, on la maintient 1 h. Le mélange est encore agité, à 20°C, durant 3 h.

La solution brune, visqueuse, est diluée par 150 cm³ d'acétate d'éthyle, puis agitée 30 min avec 20 g de charbon végétal. Elle est filtrée, lavée par décantation, avec de l'eau (3×150 cm³), puis séchée ($Na_2SO_4$) et filtrée.

Le solvant est évaporé sous vide, le résidu visqueux est agité avec 250 cm³ d'oxyde d'isopropyle. Les cristaux sont essorés et séchés (Rendement : 166 g). Ils sont recristallisés dans 800 cm³ d'un mélange acétate d'éthyle (1 volume) - oxyde d'isopropyle (1 volume). On obtient 121 g (64%) de [benzimidazolyl-1]-1 oxo-3 butane (IX), F. 64°C.

| *Analyse* pour $C_{11}H_{12}N_2O$ (188,22) | | | | |
|---|---|---|---|---|
| | Calculé % | C 70,18 | H 6,42 | N 14,88 |
| | Trouvé % | C 69,97 | H 6,62 | N 14,88 |

260 g (1,38 mole) de cette cétone, en solution dans 900 cm³ d'éthanol sont agités dans un ballon de 5 litres muni d'un réfrigérant à reflux.

On ajoute une solution d'acétate d'hydroxylamine préparée à partir de 191,8 g (2,76 moles) de chlorhydrate d'hydroxylamine, 900 cm³ d'eau et 375 g (2,76 moles) d'acétate de sodium (trihydrate).

Le mélange est agité et chauffé 2 h à reflux. Après retour à la température ordinaire, l'oxime cristallise. Elle est essorée, lavée à l'eau puis à l'éthanol et séchée à l'air. On obtient 214 g (Rendement 77%) de [benzimidazolyl-1]-1 oximino-3 butane (X), F.=144–146°C, après recristallisation dans l'éthanol à 50%. F. 148–150°C.

| *Analyse* pour $C_{11}H_{13}N_3O$ (P.M. 203,24) | | | | |
|---|---|---|---|---|
| | Calculé % | C 65,00 | H 6,45 | N 20,68 |
| | Trouvé % | C 64,90 | H 6,15 | N 20,91 |

Dans un autoclave de 1 litre, 107 g (0,53 mole) d'oxime sont mis en suspension dans 300 cm³ d'éthanol ; on ajoute 50 cm³ d'une solution d'ammoniaque à 34% et 35 g de nickel de Raney. Le mélange est agité en atmosphère d'hydrogène, sous une pression initiale de 80 bars, à 70–80°C. Après 5 heures, l'opération est terminée. Le catalyseur est séparé par filtration. La solution est concentrée à sec sous vide ; le résidu visqueux repris par 150 cm³ d'eau et 80 cm³ de lessive de soude. La base qui se sépare est extraite au chloroforme (3×150 cm³). La solution organique est séchée ($MgSO_4$). Le solvant est évaporé et le résidu fractionné sous vide.

On obtient 76,7 g (Rendement 77%) d'amino-3 [benzimidazolyl-1]-1 butane, $E_{0,7}$ : 164–168°C, hygroscopique.

*Spectre de R.M.N.* ($CDCl_3$)- δ=7,35 ppm, m, 2H et δ=6,85 ppm, m,

3H (aromatiques + 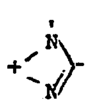 H ), δ = 4,0 ppm, t, 2H ( 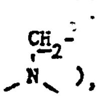 ), J = 7 Hz ;

δ=2,66 ppm, m. 1H (-CH -$CH_3$) ; δ=1,70 ppm, m, 2H (-$CH_2$-$CH_2$-CH-) ; δ=1 ppm, 5H (-$CH_3$+$NH_2$), 2H, effaçables par $D_2O$.

b) *Ethoxy-2 hydroxy-2 (méthoxy-3 phénylméthoxy-4 phényl)-1 éthanone*

79,4 g (0,237 mole) de bromo-2 (méthoxy-3 phénylméthoxy-4 phényl)-1 éthanone et 21,1 g (0,237 mole) de diéthylhydroxylamine dans 610 cm³ de méthanol sont agités et chauffés à reflux durant 8 h.

La bromocétone passe en solution durant la première heure.

Le méthanol est chassé sous vide, et le résidu est repris par 500 cm³ de toluène.

Le bromhydrate de diéthylamine, insoluble, est séparé par filtration.

La solution toluénique est lavée à l'eau jusqu'à ce que les extraits aqueux ne contiennent plus d'ions Br-. Après séchage ($MgSO_4$), la solution organique est concentrée à sec sous vide. Le résidu, coloré en rouge, est dissous dans 140 cm³ d'éthanol et la solution est abandonnée pendant 24 h à la température ordinaire. Le solide qui a cristallisé est essoré et recristallisé dans 160 cm³ d'éthanol. On obtient 53 g (Rendement 71%) d'hémiacétal, F. 98–100°C.

| *Analyse* pour $C_{18}H_{20}O_5$ (P.M. 316,34) | | | |
|---|---|---|---|
| | Calculé % | C 68,34 | H 6,37 |
| | Trouvé % | C 68,57 | H 6,56 |

*Spectre de R.M.N.* (D.M.S.O.$d_6$)

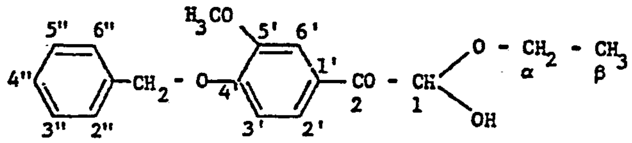

δ=7,25 ppm ; d (dédoublé), 1H ($H_{2'}$) ; δ=7,10 ppm, d, 1H ($H_{6'}$) J ($H_{2'}$–$H_{6'}$)=2 Hz ; δ=6,95 ppm, m, 5H (aromatiques : 2"+3"+4"+5"+6") ; δ=6,7 ppm, d, 1H ($H_{3'}$), J ($H_{2'}$–$H_{3'}$)=8 Hz ; δ=6,30 ppm, d, 1H (échangeable par $D_2O$) (OH) ; δ=5,12 ppm, d, 1H ($H_1$), J (OH–$H_1$)=8 Hz ; δ=4,82 ppm, s. 2H (–$CH_2O$) ;δ=3,57 ppm, s, 3H (–$CH_3O$) ; δ=3,5 ppm, q, 2H (–$CH_2\alpha$) ; δ=1,05 ppm, t (–$CH_3\beta$) J [$CH_2\alpha$–$CH_3\beta$]=7 Hz.

***Exemple 2***

*Dichlorhydrate de [benzimidazolyl-1]-1, N-[(hydroxy-4 méthoxy-3 phényl)-2 hydroxy-2 éthyl] amino-3 butane, dihydrate* (II, A=C1, n=1, n'=1, n"=2)

a) On suit le mode opératoire décrit à l'exemple 1 ; 38 g (0,2 mole) d'amino-3 [benzimidazolyl-1]-1 butane, en solution dans 550 cm³ d'éthanol, sont additionnés de 56 g de (méthoxy-3 phénylméthoxy-4) phénylglyoxal (hémihydrate), et le mélange est agité pendant 3 h à la température ordinaire.

La solution est refroidie à 10°C, puis additionnée de 15,2 g (0,4 mole) de borohydrure de sodium. Lorsque la réduction est terminée, le milieu est acidifié par 40 cm³ d'acide méthanesulfonique. Après agitation pendant 1 h à 1a température ordinaire, on ajoute 100 cm³ d'eau et concentre sous vide au 1/3 du volume ; le résidu est repris par 300 cm³ d'eau. Après extraction à l'acétate d'éthyle, la solution est rendue alcaline par addition de lessive de soude ; la base est extraite avec de l'acétate d'éthyle (200 cm³+100 cm³+100 cm³). Après séchage ($MgSO_4$) et concentration sous vide, on obtient 89 g de base brute.

Elle est dissoute dans 150 cm³ d'éthanol et agitée pendant 1 h avec 35 g de nickel de Raney. La solution est filtrée, chauffée à 70°C, et additionnée de 49 cm³ d'acide chlorhydrique alcoolique 8, 15N, sous agitation. Un précipité initialement formé passe en solution, en fin d'addition. Après retour à température ordinaire, (début de cristallisation), le mélange est abandonné pendant une nuit au réfrigérateur. Les cristaux sont essorés, lavés à l'éthanol, puis à l'acétone et séchés sous vide phosphorique.

On obtient ainsi 56 g d'un monohydrate du dichlorhydrate de [benzimidazolyl-1]-1 N-[(méthoxy-3 phénylméthoxy-4 phényl)-2 hydroxy-2 éthyl] amino-3 butane. F. ∼ 190°C (pâteux).

| | | | | | |
|---|---|---|---|---|---|
| *Analyse* pour $C_{27}H_{31}O_3N_3$, 2 HC1, $H_2O$ (P.M. 536,49) | Calculé % | C 60,44 | H 6,58 | N 7,83 | Cl 13,22 |
| | Trouvé % | C 60,95 | H 6,34 | N 7,94 | Cl 13,26 |

b) On procède comme décrit à l'exemple 1 ; 20 g de ce dichlorhydrate, en suspension dans 250 cm³ d'éthanol à 50% sont hydrogénés sous pression (20 bars), en présence de 2 g de charbon palladié 10%, à la température ordinaire.

Après filtration, la solution est concentrée à sec au Rotavapor, le résidu est repris par 150 cm³ d'éthanol, et concentré à nouveau à sec. Le résidu visqueux est dissous dans 95 cm³ d'éthanol à 95%. La solution est agitée jusqu'à début de cristallisation. Après un repos de 24 h à la température ordinaire, le sel est essoré puis recristallisé dans 56 cm³ d'éthanol. Il est séché pendant une nuit dans le vide phosphorique, puis laissé à l'air jusqu'à poids constant (reprise d'humidité). On obtient 10,8 g d'un hydrate solvaté par une molécule d'éthanol.

| | | | | | |
|---|---|---|---|---|---|
| *Analyse* pour $C_{20}H_{25}N_3O_3$, 2HC1, $H_2O$, $C_2H_5OH$ (P.M. 492,44) | Calculé % | C 53,66 | H 7,16 | N 8,53 | Cl 14,40 |
| | Trouvé % | C 53,49 | H 7,22 | N 8,85 | Cl 14,24 |

La présence de l'alcool de solvatation est confirmée par le spectre de R.M.N.

*Spectre de R.M.N.* ($D_2O$)

δ = 9,25 ppm, s, 1H (-N=CH-N-) ; δ = 6,7 à 8 ppm, m, 7H (aromatiques);

δ = 4,95 ppm, t, 1H, ($CH_2$ - $CH_2$ - CH(OH) -), J($CH_2$-CH-) = 6Hz ;

δ = 4,7 ppm, m, (-N - $CH_2$- + CH($CH_3$)-) ; δ = 3,82 ppm, s, 3H (-$OCH_3$) ;

δ=3,67 ppm, q, 2H ($CH_3$–$CH_2$–OH), J=7 Hz ; δ=3,3 ppm, d, 2H (NH–$CH_2$–) ; δ=2,5 ppm, q, 2H (–N–$CH_2$–$CH_2$–) ; δ=1,55 ppm, d, 3H, $CH_3$ (–CH–) ; δ=1,22 ppm, t, 3H, ($CH_3$–$CH_2$OH), J=7 Hz.

L'hémihydrate du (méthoxy-3 phénylméthoxy-4) phénylglyoxal, nécessaire à cette préparation, est obtenu comme il est décrit ci-dessous :

44,4 g d'anhydride sélénieux (0,4 mole), 350 cm³ de dioxanne et 10 cm³ d'eau sont agités et chauffés à reflux jusqu'à dissolution du dioxyde de sélénium.

Après retour à 80°C, 102,5 g (0,4 mole) de (méthoxy-3 phénylméthoxy-4)-1 éthanone sont ajoutés, et le mélange est agité et chauffé à reflux jusqu'à disparition complète de la cétone de départ (mise en évidence par C.C.M. sur silicagel, F. 257 MERCK ; éluant : chloroforme : 95 volumes, méthanol : 5 volumes), ce qui demande de 8 à 10 h.

Après refroidissement, le sélénium formé est séparé par filtration, la solution est concentrée à sec sous vide. Le

résidu est dissous dans 150 cm³ de toluène et 100 cm³ d'eau sont ajoutés sous agitation. L'hémihydrate cristallise lentement. Après une nuit de repos, à la température ordinaire, il est essoré, puis dissous à reflux dans 150 cm³ de toluène additionné de 20 cm³ d'eau. La solution chaude est filtrée sur cellite (élimination de particules de sélénium). Les cristaux qui précipitent par refroidissement sont essorés et séchés à l'air. On obtient 90,4 g d'un hémihydrate, F. peu net ~ 140°C.

*Analyse* pour $C_{16}H_{14}O_4$, 0,5 $H_2O$ (P.M. 279,27)

| | | |
|---|---|---|
| Calculé % | C 68,81 | H 5,41 |
| Trouvé % | C 68,80 | H 5,59 |
| | 68,85 | 5,63 |

**Exemple 3**

*Sulfate neutre de [benzimidazolyl-1]-1, N-[(hydroxy-4 méthoxy-3 phényl)-2 hydroxy-2 éthyl] amino-3 butane-monohydrate* (II, A=$SO_4$, n=1, n'=2, N''=1)

16,5 g de [benzimidazolyl-1]-1 N-[(méthoxy-3 phénylméthoxy-4 phényl)-2 hydroxy-2 éthyl] amino-3 butane brut, obtenu selon l'exemple 1, sont dissous dans de l'acide acétique, et le volume ramené à 100 cm³. 1 cm³ de la solution est prélevé et la basicité est dosée par l'acide perchlorique 0,105 N. A partir de ce résultat, on calcule la quantité d'acide sulfurique 2N (en solution acétique) nécessaire pour neutraliser la totalité des bases (soit 31,5 cm³, pour l'exemple cité). Ce volume de réactif est ajouté à la solution de base, le solvant est chassé par évaporation au Rotavapor, le résidu est repris par 50 cm³ d'eau qui sont évaporés dans le même appareil. Cette opération est répétée une nouvelle fois, afin d'éliminer l'acide acétique aussi complètement que possible. Le résidu est repris par 75 cm³ d'eau et abandonné à la température ambiante durant 24 h. Le sel qui a cristallisé est essoré, lavé á l'eau glacée et séché dans le vide phosphorique. On obtient 15,8 g de sulfate neutre de [benzimidazolyl-1]-1 N-[méthoxy-3 phénylméthoxy-4 phényl)-2 hydroxy-2 éthyl] amino-3 butane, F. ~ 170–180°C.

*Analyse* pour $C_{27}H_{31}N_3O_3$, $H_2SO_4$ (P.M. 543,56)

| | | | |
|---|---|---|---|
| Calculé % | C 59,66 | H 6,12 | N 7,73 |
| Trouvé % | C 59,40 | H 6,39 | N 7,85 |

15,7 g de ce sulfate, en suspension dans 250 cm³ d'éthanol à 50%, sont hydrogénés en présence de 1,6 g de Pd à 10% sur charbon, comme décrit à l'exemple 1, à 40°C, à la pression ordinaire pendant une durée de 4 h. Après filtration, la solution est concentrée à sec au Rotavapor, le résidu est repris par 100 cm³ d'éthanol et concentré à nouveau. Cette évaporation est répétée, pour éliminer l'eau au maximum. Le résidu est repris par 100 cm³ d'éthanol et abandonné pendant 24 h à la température ordinaire. Le sel qui a cristallisé est essoré et séché (13 g). Il est recristallisé dans 80 cm³ d'éthanol à 70%. Après essorage, séchage sous vide phosphorique et reprise d'humidité à l'air, on obtient 8,5 g de sulfate neutre de [benzimidazolyl-1]-1 N-[(hydroxy-4 méthoxy-3 phényl)-2 hydroxy-2 éthyl] amino-3 butane, monohydrate solvaté par 1/2 molécule d'éthanol.

*Analyse* pour $C_{20}H_{25}N_3O_3$, $H_2O$, $H_2SO_4$, 0,5 $C_2H_5OH$ (P.M. 494,55)

| | | | | |
|---|---|---|---|---|
| Calculé % | C 50,99 | H 6,52 | N 8,50 | S 6,48 |
| Trouvé % | C 50,90 | H 6,38 | N 8,43 | S 6,54 |

La présence de la demi-molécule d'éthanol est confirmée par le spectre de R.M.N.

**Exemple 4**

Fumarate de [benzimidazolyl-1]-1, N-[hydroxy-4 méthoxy-3 phényl)-2 hydroxy-2 éthyl] amino-3 butane.

1) [benzimidazolyl-1]-1, N[(hydroxy-4 méthoxy-3 phényl) 2 hydroxy-2 éthyl] amino-3 butane.

2 g du dichlorhydrate hydraté et solvaté, préparé selon l'exemple 2, sont dissous dans 10 cm³ d'eau ; la solution est rendue alcaline par addition de 10 cm³ d'une solution saturée de carbonate de sodium. La base visqueuse qui précipite est extraite par le dichlorométhane (20 cm³+3×15 cm³). Les phases organiques, réunies, sont séchées ($MgSO_4$), et le solvant est évaporé sous vide.

Le [benzimidazolyl-1]-1, N-[(hydroxy-4 méthoxy-3 phényl)-2 hydroxy-2 éthyl] amino-3 butane ainsi obtenu est une huile visqueuse dont le spectre de R.M.N. confirme la structure annoncée :

<u>Spectre de R.M.N.</u>($CDCl_3$) : δ = 7,75 ppm, s, 1H 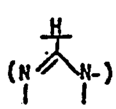 ; δ = 7,75 à 7,6 ppm, m, 1 H arom. ; δ= 7,35 à 7,1 ppm, m, 3H arom. ( 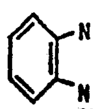 ) ; δ = 6,9 à 6,7 ppm, m, 3H arom. 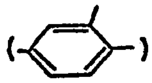 ; δ = 4,55 ppm, m, 1H (-CH-) (OH) ;

δ = 4,15 ppm, t, 2H ( 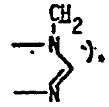 ), J = 7Hz ; δ = 3,92 ppm, m, 3H (2 OH + NH) ; δ = 3,75 ppm, s, 3H ($-OCH_3$) ; δ = 2,3 à 2,9 ppm, m, 3H ($NH - CH_2 + -CH(CH_3)-$ ) ; δ = 1,80 ppm, q, 2H ($-N - CH_2 - CH_2-$), J = 7Hz ; δ = 1,02 ppm, d, 3H ($-CH(CH_3)-$), J = 6,5 Hz.

2) Sel fumarate.

Le résidu (1,3 g) est dissous dans 10 $cm^3$ d'éthanol ; on ajoute 0,46 g d'acide fumarique. La suspension est chauffée à reflux jusqu'à dissolution. Par refroidissement, le sel cristallise. Il est essoré et recristallisé 10 $cm^3$ d'éthanol à 90%. On obtient 1,2 g (Rendement 69,5%) du sel, F. 200°C.

| | | | | |
|---|---|---|---|---|
| *Analyse* pour $C_{20}H_{25}N_3O_3$, HOOCCH=CH-COOH (P.M. 471,50) | Calculé % | C 61,13 | H 6,20 | N 8,91 |
| | Trouvé % | C 60,78 | H 6,81 | N 8,73 |

Les composés selon l'invention ont fait l'objet d'une étude pharmacologique dans les principaux domaines d'activité des β-mimétiques.

Cette étude a été réalisée notamment avec le composé de l'exemple 1 et celui de l'exemple 2.

Dans la présentation des expériences et des résultats, le composé de l'exemple 1 est appelé : composé A et le composé de l'exemple 2 est appelé : composé B.

Ces deux composés ont fait l'objet d'une étude de toxicité aiguë chez la souris.

Par voie intraveineuse, le composé A présente une $DL_{50}$ de 114 mg/kg (limites de confiance, à p=0,05 : 102–128) soit, exprimé en base : 96 mg/kg (85–107) ; le composé B présente une $DL_{50}$ de 148 mg/kg (limites de confiance, à p=0,05 : 130–168) soit, exprimé en base : 106 mg/kg (94–121).

Les deux composés ont une toxicité analogue et l'étude pharmacologique a pu, indifféremment, être réalisée avec l'un ou l'autre, les résultats étant, dans les deux cas, exprimés en poids de base par kg de poids corporel pour les essais *in vivo* et en concentration molaire pour les essais *in vitro.*

L'étude pharmacologique a été réalisée comparativement au Salbutamol (sulfate) qui est un stimulant β-adrénergique et un agent bronchodilatateur du commerce, pour lequel les résultats ont été exprimés de même en poids de base par kg de poids corporel ou en concentration molaire.

## ACTIVITE RELAXANTE DE LA TRACHEE OU DES BRONCHES

1.1. *Activité relaxante du muscle trachéal, in vitro*

Cette activité est étudiée sur une préparation destrachée isolée de cobaye (chaîne d'anneaux trachéaux, selon CASTILLO et de BEER. Journ. Pharmacol. Exp. Ther. 1947 *90* 104–109), maintenue en survie dans une solution de KREBS. L'effet de doses aumulées sur la tension de la préparation est enregistré.

L'activité de la substance est exprimée par la valeur de la $CD_{50}$ molaire (concentration décontracturante 50=concentration donnant 50% de la relaxation maximale), dont le log, négatif est la valeur $pD_2$, et par l'activité intrinsèque correspondant à l'effet maximal.

Ces valeurs ont été déterminées parallèlement pour le composé A et le Salbutamol, sur 6 préparations pour chacun.

| | COMPOSE A | SALBUTAMOL |
|---|---|---|
| CD 50 | $7,1\times10^{-8}$ mol | $1,5\times10^{-8}$ mol |
| $PD_2$ | 7,15 | 7,82 |
| Réponse maximale (décontraction) | 390 mg | 358 mg |
| Activité intrinsèque | 1,09 | 1,00 |

L'activité du composé A sur le muscle trachéal est très voisine de celle du Salbutamol.

1.2. *Activité bronchodilatatrice, in vivo*

1.2.1 *Chez le Cobaye anesthésié*

(méthode de KONZETT. Arch. Exp. Pathol. Pharmakol. 1940 *195* 71–74).

Un bronchospasme est provoqué par injection intraveineuse d'histamine. L'activité bronchodilatatrice est montrée par inhibition partielle ou totale de ce spasme.

Le produit étudié été administré par voie intraveineuse ou par voie intraduodénale et l'histamine injectée ensuite à différents temps pour suivre le délai d'apparition et la durée de l'effet inhibiteur du traitement.

1.2.1.1. *Traitement par voie intraveineuse*

Les résultats ci-dessous sont les moyennes des valeurs obtenues sur 10 animaux.

| Dose µg base/kg | COMPOSE A % inhibition aux temps (min.) | | | | | | |
|---|---|---|---|---|---|---|---|
| | 1 | 5 | 10 | 15 | 30 | 45 | 60 |
| 0,08 | 1 | 1 | 0 | 0 | | | |
| 0,25 | 9 | 1 | 0 | 0 | | | |
| 0,8 | 36 | 3 | 2 | 3 | | | |
| 2,5 | 66 | 13 | 8 | 8 | | | |
| 8 | 73 | 17 | 12 | 8 | | | |
| 25 | 92 | 52 | 38 | 28 | | | |
| 84 | 92 | 80 | 70 | 63 | 51 | 36 | 21 |

| Dose µg base/kg | SALBUTAMOL % inhibition aux temps (min.) | | | | | | | |
|---|---|---|---|---|---|---|---|---|
| | 1 | 5 | 10 | 15 | 30 | 45 | 60 | |
| 0,08 | 8 | 2 | 0 | 1 | | | | |
| 0,25 | 25 | 6 | 2 | 2 | | | 0,8 | 54 |
| 8 | 7 | 2 | | | | | | |
| 2,5 | 75 | 13 | 8 | 4 | | | | |
| 8 | 91 | 35 | 13 | 8 | | | | |
| 25 | 97 | 78 | 34 | 22 | | | | |
| 83 | 94 | 81 | 60 | 55 | 50 (n=9) | 30 | 17 (n=9) | |

Par voie intraveineuse, le composé A et le Salbutamol ont une activité de même ordre, quoique très légèrement inférieure pour le composé A aux doses faibles ; à la dose la plus élevée, dont l'effet est intense et durable, le composé A présente une activité au moins égale à celle du Salbutamol.

1.2.1.2. *Traitement par voie intraduodénale*

Les résultats ci-dessous sont les moyennes des valeurs obtenues sur 10 animaux.

| Dose mg base/kg | COMPOSE A % inhibition aux temps (min.) | | | | | | | |
|---|---|---|---|---|---|---|---|---|
| | 1 | 5 | 15 | 30 | 45 | 60 | 90 | 120 |
| 0,25 | 11 | 27 | 26 | 26 | 19 | 13 | 9 | – |
| 0,8 | 16 | 60 | 61 | 44 | 29 | 23 | 21 | – |
| 2,5 | 20 | 73 | 65 | 53 | 44 | 39 | 34 | 30 |
| 8 | 51 | 82 | 82 | 71 | 67 | 65 | 59 | 55 (n=9) |

| Dose mg base/kg | SALBUTAMOL % inhibition aux temps (min.) | | | | | | | |
|---|---|---|---|---|---|---|---|---|
| | 1 | 5 | 15 | 30 | 45 | 60 | 90 | 120 |
| 0,8 | 4 | 18 | 16 | 16 | 13 | 13 | - | - |
| 2,5 | 2 | 14 | 32 | 34 | 29 | 23 | 15 | 10 |
| 8 | 31 | 64 | 76 | 72 | 65 | 58 | 52 | 37 (n=8) |

La dose seuil d'activité du composé A se situe à 0,25 mg/kg ; l'effet devient intense entre 0,8 et 2,5 mg/kg et plus durable à 8 mg/kg.

Le Salbutamol a une dose seuil comprise entre 0,8 et 2,5 mg/kg (plus proche de 2,5) ; il faut atteindre 8 mg/kg pour obtenir une activité forte, comprise entre celles montrées respectivement par 2,5 et 8 mg/kg de composé A.

Par cette voie, le composé A est plus actif que le Salbutamol.

1.2.2. *Chez le chien anesthésié*

(enregistrement de la pression intratrachéale)

Un bronchospasme est provoqué par injection intraveineuse d'histamine. L'activité brochodilatatrice est montrée par inhibition partielle ou totale de ce spasme.

Les produits étudiés ont été administrés par voie intraduodénale et l'histamine injectée ensuite à différents temps pour suivre l'effet du traitement.

Les résultats portés sur le tableau ci-dessous sont les moyennes des valeurs obtenues chez 3 animaux pour chaque produit.

| Dose mg base/kg | COMPOSE A % inhibition aux temps (min.) | | | | |
|---|---|---|---|---|---|
| | 5 | 20 | 35 | 60 | 90 |
| 0,025 | 48 | 60 | 67 | 63 | 62 |
| 0,08 | 70 | 78 | 76 | 70 | 60 |

| Dose mg base/kg | SALBUTAMOL % inhibition aux temps (min.) | | | | |
|---|---|---|---|---|---|
| | 5 | 20 | 35 | 60 | 90 |
| 0,025 | 21 | 65 | 63 | 61 | 65 |
| 0,08 | 8 | 50 | 56 | 55 | 52 |

Les deux produits ont une activité équivalente à 0,025 mg/kg mais le composé A agit plus rapidement (inhibition déjà forte à 5 minutes).

A la dose de 0,08 mg/kg, le composé A, dont l'activité est supérieure ou égale à celle du Salbutamol, est caractérisé par sa grande rapidité d'action.

## ACTIVITE RELAXANTE DE L'UTERUS

2.1. *In vitro*

Cette activité est étudiée sur une préparation d'utérus de rate, mise en oestrus par injection sous-cutanée de benzoate d'oestradiol. Les cornes utérines sont maintenues en survie dans une solution de RINGER-LOCKE, modifiée par GADDUM et VOGT.

L'effet relâchant du composé A et du Salbutamol est étudié vis-à-vis de contractions provoquées par de l'acétylcholine ou de la sérotonine, par l'addition préalable de concentrations croissantes, isolées ; ces composés ont un effet inhibiteur proportionnel à la concentration utilisée.

Leur activité est exprimée par la valeur de la $CE_{50}$ molaire (concentration efficace 50, inhibant de 50% la contraction témoin induite par l'agent contracturant).

Chaque $CE_{50}$ indiquée ci-dessos est obtenue à partir des résultats de 7 préparations.

| Agent contracturant | $CE_{50}$ mol Composé A | Salbutamol |
|---|---|---|
| Acétylcholine | $6{,}7\times10^{-10}$ | $5{,}0\times10^{-9}$ |
| Sérotonine | $2{,}4\times10^{-9}$ | $8{,}3\times10^{-9}$ |

L'activité du composé A sur l'utérus est de même ordre que celle du Salbutamol.

2.2. *In vivo*

L'essai est réalisé également chez le rat.

L'animal est anesthésié par du carbamate d'éthyle.

Les contractions provoquées d'une corne utérine extériorisée et immergée dans un bain nourricier sont enregistrées (bain : RINGER-LOCKE modifié par GADDUM et VOGT). Ces contractions sont induites par l'injection intraveineuse d'ocytocine, à la dose de 0,1 UI par kg.

Après un enregistrement témoin de l'effet de cette dernière, le composé A ou le Salbutamol sont administrés par voie intraveineuse et une nouvelle injection d'ocytocine est pratiquée ; son effet est comparé à celui de la réaction témoin.

Le composé A, administré à la dose de 16,8 µg/kg a une activité inhibitrice comparable à celle du salbutamol, administré à la dose de 24,9 µg/kg.

## 3. Activite cardiovasculaire

3.1. *Activité sur la fréquence cardiaque, in vitro*

Cette activité est étudiéesur une préparation d'oreillette droite isolée de cobaye maintenue en survie dans une solution de RINGER. L'effet de doses cumulées sur la fréquence des battements est enregistréa. Les résultats ci-dessous sont les moyennes de 6 préparations pour le composé A, de 4 pour le Salbutamol

| Produit | Concentration mol | Fréquence des battements (coups par min) basale | maximale | Augmentation |
|---|---|---|---|---|
| | $1\times10^{-7}$ | 209 | 249 | +40 |
| Composé A | $1\times10^{-6}$ | 209 | 258 | +49 (maxi) |
| | $1\times10^{-5}$ | 209 | 256 | +47 |
| | $1\times10^{-8}$ | 202 | 251 | +49 |
| | $1\times10^{-7}$ | 202 | 292 | +90 |
| Salbutamol | $1\times10^{-6}$ | 202 | 308 | +106 |
| | $1\times10^{-5}$ | 202 | 317 | +115 (maxi) |
| | $1\times10^{-4}$ | 202 | 312 | +110 |

Le composé A augmente la fréquence mais l'effet maximal est très inférieur à celui du Salbutamol ; cette faible activité empêche de calculer un $CE_{50}$ et une $pD_2$ pour le composé A (maximum inférieur à 50% du maximum de tachycardie obtenue avec l'isoprénaline sur la même préparation, en début d'essai).

Le composé A, tout en présentant l'activité tachycardisante caractéristique des β-mimétiques, n'a qu'un effet partiel, très inférieur à celui du Salbutamol.

### 3.2. Activité in vivo

3.2.1. *Activité sur la fréquence cardiaque et sur la pression aortique moyenne du chien anesthésié*

L'activité du composé A et du Salbutamol a été étudiée sur 2 lots de 3 chiens par produit : l'un en respiration spontanée, l'autre en respiration artificielle ; les deux substances ont été administrées par voie intraduodénale à 3 doses : 0,008–0,025 et 0,08 mg/kg.

Le tableau ci-dessous montre les effets maximaux obtenus sur la fréquence cardiaque et la pression aortique moyenne.

| Paramètre | Type de | Effet cumulé maximal |
|---|---|---|

| enregistré | respiration | Composé A | Salbutamol |
|---|---|---|---|
| Fréquence cardiaque | Spontanée | + 17 cpm (15 min après 0,08 mg/kg) | + 26 cpm (20 min après 0,08 mg/kg) |
| | Artificielle | + 20 cpm (10 min après 0,08 mg/kg) | + 41 cpm (15 min après 0,025 mg/kg) |
| Pression aortique moyenne | Spontanée | – 15 mmHg (20 min après 0,08 mg/kg) | – 40 mmHg (1 h après 0,08 mg/kg) |
| | Artificielle | – 26 mmHg (75 min après 0,08 mg/kg) | – 36 mmHg (90 min après 0,08 mg/kg) |

Le composé A est un peu moins tachycardisant et hypotenseur que le Salbutamol.

3.2.2. *Activité sur la fréquence cardiaque du chien non anesthésié*

L'expérience a porté sur 12 chiens Beagle répartis en 3 groupes de 4 ; chaque groupe a été traité, par voie orale, à 2 doses de composé B et de Salbutamol, avec un temps de repos de plusieurs jours entre les essais.

Chaque moyenne notée dans le tableau ci-dessous porte sur les 12 chiens.

| | COMPOSE B | | | | SALBUTAMOL | | | |
|---|---|---|---|---|---|---|---|---|
| Temps | 22 µg base/kg | | 72 µg base/kg | | 25 µg base/kg | | 83 µg base/kg | |
| | moy. | variat. | moy. | variat. | moy. | variat. | moy. | variat. |
| 0 | 94 | - | 90 | - | 92 | - | 90 | - |
| 15 min | 130 | + 36 | 147 | + 57 | 142 | + 50 | 163 | + 73 |
| 30 min | 129 | + 35 | 162 | + 72 | 165 | + 73 | 194 | + 104 |
| 1 h | 129 | + 35 | 162 | + 72 | 170 | + 78 | 197 | + 107 |
| 2 h | 119 | + 25 | 160 | + 70 | 156 | + 64 | 195 | + 105 |
| 3 h | 109 | + 15 | 146 | + 56 | 137 | + 45 | 172 | + 82 |
| 4 h | 104 | + 10 | 132 | + 42 | 130 | + 38 | 150 | + 60 |
| 5 h | 102 | + 8 | 125 | + 35 | 120 | + 28 | 148 | + 58 |
| 6 h | | | 115 | + 25 | | | 141 | + 51 |
| 7 h | | | 112 | + 22 | | | 138 | + 48 |

Les valeurs encadrées correspondent à l'action maximale observée.

A un niveau de dose équivalent, le Salbutamol est plus actif que le composé B : l'effet de la dose faible de Salbutamol est égal à celui de la dose forte du composé B (respectivement 25 et 72 µg/kg).

Le composé B est donc nettement moins tachycardisant que le Salbutamol chez le chien éveillé.

## 4. Specificite β-stimulante

La spécificité β-stimulante a été prouvée dans deux domaines :

– respiratoire (trachée de cobaye, in vitro)

– cardiovasculaire (fréquence cardiaque du chien, in vivo) par l'effet antagoniste d'un produit β-bloquant.

4.1. *Trachée de cobaye*

Les $CD_{50}$ avant et après addition de propranolol ($1{,}7 \times 10^{-8}$ mol=dose seuil) ont été déterminées pour le composé A et pour le Salbutamol (6 préparations par produit).

Pour les deux produits, la $CD^{50}$ a été multipliée par 3,5 sous l'effet inhibiteur du propranolol.

| | $CD_{50}$ mol | | Rapport |
|---|---|---|---|
| Produit | Avant propranolol | Après propranolol | des $CD_{50}$ |

| | | | |
|---|---|---|---|
| Composé A | $7{,}1\times10^{-8}$ | $2{,}5\times10^{-7}$ | 3,5 |
| Salbutamol | $1{,}1\times10^{-8}$ | $3{,}8\times10^{-8}$ | 3,5 |

4.2. *Fréquence cardiaque du chien anesthésié*

L'acébutolol (β-bloquant cardiosélectif), à la dose de 0,5 mg/kg par voie intraveineuse, antagonise complètement la tachycardie provoquée par le composé A, perfusé pendant 6 minutes à la dose de 4,2 µg/kg et par min.

Les résultats ci-dessous sont les moyennes des valeurs obtenues chez 3 chiens.

| Temps | | Fréquence cardiaque | Variation |
|---|---|---|---|
| T 0 | | 157 | – |
| T 0+1 min | | 180 | + 23 |
| T 0+2 min | Perfusion | 197 | + 40 |
| T 0+4 min | composé A | 202 | + 45 |
| T 0+6 min | | 205 | + 48 |
| =T 1 Inj. acébutolol | | | |
| T 1+1 min | | 162 | + 5 |
| T 1+5 min | | 156 | − 1 |
| T 1+15 min | | 149 | − 8 |
| T 1+30 min | | 147 | − 10 |
| T 1+1 h | | 154 | − 3 |
| T 1+4 h | | 143 | − 14 |
| T 1+4 h 1 min | | 146 | − 11 |
| T 1+4 h 2 min | Perfusion | 155 | − 2 |
| T 1+4 h 4 min | composé A | 160 | + 3 |
| T 1+4 h 6 min | | 160 | + 3 |

En conclusion, les résultats rapportés ci-dessus montrent que les composés, étudiés en comparaison avec le Salbutamol, possèdent :

– une activité relaxante de la trachée isolée similaire,

– une activité bronchodilatatrice comparable par voie intraveineuse, supérieure ou égale et d'induction plus rapide par voie intraduodénale,

– une activité utérorelaxante comparable, *in vitro* et *in vivo,*

– une activité tachycardisante, *in vitro,* très inférieure,

– une activité tachycardisante par voie orale inférieure chez l'animal anesthésié et surtout chez l'animal non anesthésié.

Cet ensemble de résultats permet d'envisager l'utilisation thérapeutique des composés, leur sélectivité sur les récepteurs adrénergiques bronchiques et utérins ($\beta_2$) par rapport aux récepteurs cardiaques ($\beta_1$) se montrant supérieure à celle du Salbutamol.

De plus, la rapidité d'apparition de l'effet bronchodilatateur par voie entérale, jointe à une durée d'action satisfaisante, contribue à l'intérêt de ces composés.

## Patentansprüche

1. 1-(Benzimidazol-1-yl)-N-[2-(4-hydroxy-3-methoxyphenyl)-2-hydroxyäthyl]-3-aminobutan der Formel

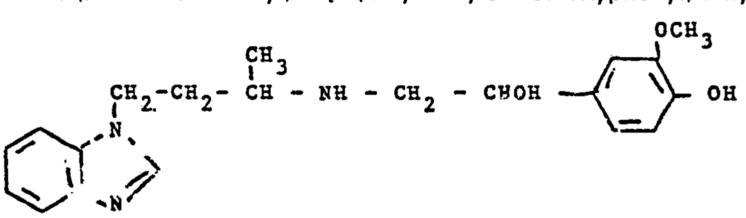

(I)

sowie seine nichttoxischen, pharmazeutisch akzeptablen anorganischen und organischen Säureadditionssalze und die Hydrate dieser Salze.

2. Basisches Sulfat von 1-(Benzimidazol-1-yl)-N-[-2-(4-hydroxy-3-methoxyphenyl)-2-hydroxyäthyl]-3-aminobutan und dessen Hydrate.

3. 1-(Benzimidazol-1-yl)-N-[2-(4-hydroxy-3-methoxyphenyl)-2-hydroxyäthyl]-3-aminobutan-dihydrochlorid und dessen Hydrate.

4. Medikament, das insbesondere zur Behandlung von Asthma oder verfrühten Wehen geeignet ist, dadurch gekennzeichnet, daß es 1-(Benzimidazol-1-yl)-N-[2-(4-hydroxy-3-methoxyphenyl)-2-hydroxyäthyl]-3-aminobutan oder ein nichttoxisches Säureadditionssalz desselben oder ein Hydrat des Salzes nach einem der Ansprüche 1 bis 3 enthält.

5. Pharmazeutische Zusammensetzung, dadurch gekennzeichnet, daß sie als aktives Ingrediens ein Medikament nach Anspruch 4 in einer medizinisch wirksamen Menge in Verbindung mit einem nichttoxischen, pharmazeutisch akzeptablen Träger oder Vehikel enthält.

6. Pharmazeutische Zusammensetzung nach Anspruch 5, dadurch gekennzeichnet, daß sie in einer für orale Verabreichung geeigneten Form, wie einer Tablette, Kapsel oder Dragée mit einer Dosis von 0,5 bis 10 mg an aktivem Ingrediens, oder als Sirup mit einer Konzentration von 0,1 bis 2 mg/cm³ an aktivem Ingrediens vorliegt.

7. Pharmazeutische Zusammensetzung nach Anspruch 5, dadurch gekennzeichnet, daß sie in einer für parenterale Verabreichung geeigneten Form, wie einer wässerigen Lösung mit einer Konzentration von 0,02 bis 0,4 mg/cm³ an aktivem Ingrediens, vorliegt.

8. Pharmazeutische Zusammensetzung nach Anspruch 5, dadurch gekennzeichnet, daß sie in Form eines Zäpfchens mit einem Gehalt von 0,25 bis 5 mg an aktivem Ingrediens pro Dosiseinheit vorliegt.

9. Verfahren zur Herstellung von 1-(Benzimidazol-1-yl)-N-[2-(4-hydroxy-3-methoxyphenyl)-2-hydroxyäthyl]-3-aminobutan der Formel

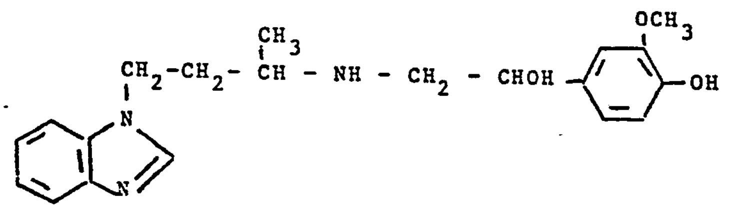 (I)

seiner pharmazeutisch akzeptablen nichttoxischen anorganischen oder organischen Säureadditionssalze und der Hydrate dieser Salze, dadurch gekennzeichnet, daß das Verfahren folgende Schritte umfaßt:

a) Kondensation von 3-Amino-1-(benzimidazol-1-yl)-butan mit (3-Methoxy-4-phenylmethoxy)-phenylglyoxal oder mit 2-Äthoxy-2-hydroxy-1-[(3-methoxy-4-phenylmethoxy)-phenyl]-äthanon in einem alkoholischen Medium zur Bildung eines Imins,

b) Reduktion dieses Imins "in situ" mit einem Überschuß an Natriumborhydrid zur Bildung von 1-(Benzimidazol-1-yl)-N-[2-hydroxy-2-(3-methoxy-4-phenylmethoxy)-phenyl-äthyl]-3-aminobutan mit anschließender Umwandlung des letzteren in ein nichttoxisches Säureadditionssalz in kristalliner Form,

c) Hydrogenolyse des Salzes zur Herstellung des entsprechenden Salzes der Verbindung (I), welches gewonnen und gegebenenfalls gereinigt wird,

d) gegebenen falls überführen des erhaltenen Salzes in die gewünschte entsprechende Base der Formal I durch Alkalisierung in einem wässerigen Medium mittels eines Alkalicarbonats oder einer schwachen Base,

e) gegebenenfalls Salzbildung der Base der Formel I mit einer nichttoxischen Säure zur Herstellung des gewünschten entsprechenden Salzes.

10. Verfahren nach Anspruch 9, dadurch gekennzeichnet, daß der Hydrogenolyseschritt in Lösung oder in Suspension in wässerigem Alkohol in Gegenwart von Palladium auf Aktivkohle und Wasserstoff bei einem Druck von 1 bis 50 bar und einer Temperatur von 20 bis 40°C durchgeführt wird.

11. Verfahren nach Anspruch 9, dadurch gekennzeichnet, daß das Überführen in ein Säureadditionssalz in Stufe b) durch Zugabe von Schwefelsäure in einer zur Salzbildung der Hälfte der basischen Funktionen des gebildeten 1-(Benzimidazol-1-yl)-N-[2-(3-methoxy-4-phenylmethoxy)-phenyl-2-hydroxyäthyl]-3-aminobutans erforderlichen Menge erfolgt, um ein basisches Sulfat zu erhalten.

12. Verfahren nach Anspruch 9, dadurch gekennzeichnet, daß das Überführen in ein Säureadditionssalz in Stufe b) durch Zugabe von Salzsäure erfolgt.

13. Verfahren nach Anspruch 9, dadurch gekennzeichnet, daß das Überführen in ein Säureadditionssalz in Stufe b) durch Zugabe von Schwefelsäure in einer zur Salzbildung der Gesamtheit der basischen Funktionen des gebildeten 1-(Benzimidazol-1-yl)-N-[2-(3-methoxy-4-phenylmethoxy)-phenyl-2-hydroxyäthyl]-3-aminobutans erforderlichen Menge zur Herstellung eines neutralen Sulfats erfolgt.

14. Verfahren nach Anspruch 9, dadurch gekennzeichnet, daß die Umwandlung des Salzes in die entsprechende Base in Stufe b) durch

– Zugabe einer gesättigten Alkalicarbonatlösung,
– Extrahieren der ausgefällten Base mit einem Lösungsmittel, wie Dichlormethan, und
– Trocknen des Extraktes und Abdampfen des Extraktionslösungsmittels unter Vakuum erfolgt.

15. Verfahren nach Anspruch 9, dadurch gekennzeichnet, daß das Ausgangs-3-Amino-1-(benzimidazol-1-yl)-butan hergestellt wird durch

– Kondensation von Benzimidazol mit einem geringen Überschuß an Methylvinylketon in Gegenwart einer katalytischen Menge an Benzyltrimethylammoniumhydroxid in einem apolaren neutralen Lösungsmittel, wie einem aromatischen Kohlenwasserstoff oder Äthylacetat, zur Bildung von 1-(Benzimidazol-1-yl)-3-oxobutan,
– Überführen des letzteren in sein Oxim mittels Hydroxylaminacetat, und

– katalytische Hydrierung des Oxims in einem alkoholischen Medium in Gegenwart von Ammoniak und Raney-Nickel bei einem Druck von 20 bis 80 bar und einer Temperatur von 50 bis 100°C zur Bildung der gewünschten Ausgangsverbindung.

16. Verfahren nach Anspruch 9, dadurch gekennzeichnet, daß das Ausgangs-(3-Methoxy-4-phenylmethoxy)-phenylglyoxal hergestellt wird durch Oxidation von 1-(3-Methoxy-4-phenylmethoxyphenyl)-äthanon mittels Selenoxid bei 100°C in einem geeigneten Lösungsmittel, wie Dioxan.

17. Verfahren nach Anspruch 9, dadurch gekennzeichnet, daß das Ausgangs-(3-Methoxy-4-phenylmethoxy)-phenylglyoxal hergestellt wird durch Behandeln des 1-(3-Methoxy-4-phenylmethoxyphenyl)-äthanons mit Brom in Gegenwart eines Niedrigalkyloxids oder mit 2-Pyrrolidonhydrotribromid in einem chlorierten aliphatischen Kohlenwasserstoff zur Bildung des entsprechenden Bromketons und anschließende Behandlung des letzteren mit N,N-Diäthylhydroxylamin.

18. Verfahren nach Anspruch 9, dadurch gekennzeichnet, daß 2-Äthoxy-2-hydroxy-1-(3-methoxy-4-phenylmethoxyphenyl)-äthanon hergestellt wird durch Behandeln von (3-Methoxy-4-phenylmethoxy)-phenylglyoxal mit Äthanol.

## Claims

1. 1-[1-benzimidazolyl[ N-]2-(4-hydroxy-3-methoxy phenyl) 2-hydroxy ethyl] 3-amino butane corresponding to the following formula :

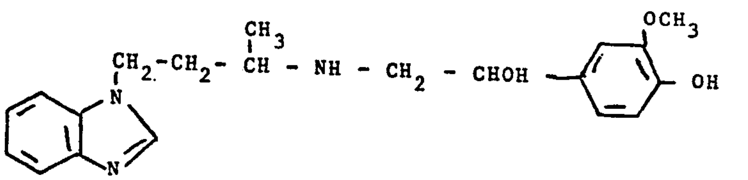

(I)

as well as its non-toxic, pharmaceutically acceptable inorganic or organic acid addition salts and the hydrates of said salts.

2. 1-[1-benzimidazolyl] N-[2-(4-hydroxy 3-methoxy phenyl) 2-hydroxy ethyl] 3-amino butane basic sulphate and the hydrates thereof.

3. 1-[1-benzimidazolyl] N-[2-(4-hydroxy 3-methoxy phenyl) 2-hydroxy ethyl] 3-amino butane dihydrochloride and the hydrates thereof.

4. Medicament useful particularly for treating asthma or premature labor characterised in that it comprises 1-[1-benzimidazolyl] N-[2-(4-hydroxy 3-methoxy phenyl) 2-hydroxy ethyl] 3-amino butane or a non-toxic acid addition salt thereof or a hydrate of said salt according to anyone of claims 1 to 3.

5. Pharmaceutical composition characterised in that it contains as active ingredient a medicament according to claim 4, associated in an effective medicinal amount with a non-toxic, pharmaceutically acceptable carrier or vehicle.

6. Pharmaceutical composition according to claim 5, characterised in that it is in a suitable form for oral administration, such as tablet, capsule or dragee, with a dosage of 0.5–10 mg of active ingredient, or syrup at a concentration of 0.1–2 mg/cm³ of active ingredient.

7. Pharmaceutical composition according to claim 5, characterised in that it is a suitable form for parenteral administration, such as aqueous solution at a concentration of 0.02 to 0.4 mg/cm³ of active ingredient.

8. Pharmaceutical composition according to claim 5, characterised in that it is in the form of a suppository containing 0.25 to 5 mg of active ingredient per dosage unit.

9. Process for preparing 1-[1-benzimidazolyl] N-[2-(4-hydroxy 3-methoxy phenyl) 2-hydroxy ethyl] 3-amino butane corresponding to the following formula :

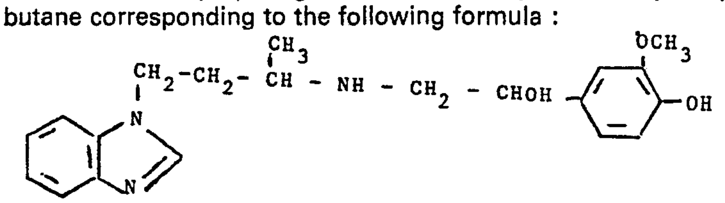

(I)

its pharmaceutically acceptable non-toxic inorganic or organic acid addition salts, and hydrates of said salts, characterised in that said process comprises the following steps :

a) condensation of 3-amino 1-[1-benzimidazolyl] butane with (3-methoxy 4-phenyl methoxy) phenylglyoxal or with 2-ethoxy 2-hydroxy 1-(3-methoxy 4-phenylmethoxy phenyl) ethanone, in an alcoholic medium, to form an imine,

b) reduction "in situ" of this imine with excess sodium borohydride to form 1-[1-benzimidazolyl] N-[2-hydroxy 2-(3-methoxy 4-phenylmethoxy phenyl) ethyl] 3-amino butane, followed by the conversion of the latter into a non-toxic acid addition salt, in crystallized form,

c) hydrogenolysis of said salt to obtain the corresponding salt of compound (I), which is recovered and optionally purified,

d) optionally transformation of the salt obtained into the desired corresponding base of formula I by alkalinisation in an aqueous medium by means of an alkali carbonate or a weak base,

e) optionally salification of the base of formula I with a non-toxic acid to obtain the desired corresponding salt.

10. Process according to claim 9, characterised in that the hydrogenolysis step is conducted in solution or in suspension in aqueous alcohol, in the presence of palladium on charcoal and hydrogen at pressure of 1 to 50 bars at a temperature of 20 to 40°C.

11. Process according to claim 9, characterised in that the transformation into an acid addition salt in step b) is done by adding sulphuric acid in an amount required to salify half of the basic functions of the formed 1-[1-benzimidazolyl] N-[2-(3-methoxy 4-phenylmethoxy phenyl) 2-hydroxy ethyl] 3-amino butane to obtain a basic sulphate.

12. Process according to claim 9, characterised in that the transformation into an acid addition salt in step b) is done by adding hydrochloric acid.

13. Process according to claim 9, characterised in that the transformation into an acid addition salt in step b) is done by adding sulphuric acid in an amount required to salify in totality the basic functions of the formed 1-[1-benzimidazolyl] N-[2-(3-methoxy 4-phenylmethoxy phenyl) 2-hydroxy ethyl] 3-amino butane to obtain a neutral sulphate.

14. Process according to claim 9, characterised in that the transformation of the salt into the corresponding base in step b) is done by :

- adding a saturated solution of an alkali carbonate,
- extracting the precipitated base by a solvent such as dichloromethame,
- drying the extract and evaporating the extraction solvent under vacuum.

15. Process according to claim 9, characterised in that the starting 3-amino 1-[1-benzimidazolyl] butane is prepared by

- condensation of benzimidazole with slight excess of methyl-vinylketone, in the presence of a catalytic amount of benzyltrimethylammonium hydroxide, in a non polar neutral solvent, such as an aromatic hydrocarbon or ethyl acetate, to form 1-[1-benzimidazolyl] 3-oxo butane,
- conversion of the latter into its oxime by hydroxylamine acetate,
- and catalytic hydrogenation of the oxime, in an alcoholic medium in the presence of ammonia and Raney nickel at pressure of 20 to 80 bars, and at a temperature of 50 to 100°C to form the desired starting compound.

16. Process according to claim 9, characterised in that the starting (3-methoxy 4-phenyl methoxy) phenylglyoxal is prepared

- by oxidation of 1-(3-methoxy 4-phenylmethoxy phenyl) ethanone by means of selenium oxide, at 100°C, in a suitable solvent, such as dioxane.

17. Process according to claim 9, wherein the starting (3-methoxy 4-phenyl methoxy) phenylglyoxal is prepared by treating 1-(3-methoxy 4-phenyl methoxy phenyl) ethanone with bromine in the presence of a lower alkyl oxide, or with 2-pyrrolidone trihydrobromide in a chlorinated aliphatic hydrocarbon to form the corresponding bromoketone, followed by treatment of the latter with N,N-diethylhydroxylamine.

18. Process according to claim 9, wherein the 2-ethoxy 2-hydroxy 1-(3-methoxy 4-phenyl methoxy phenyl) ethanone is prepared by treating (3-methoxy 4-phenyl methoxy) phenylglyoxal with ethanol.

## Revendications

1. [Benzimidazolyl-1]-1, N-[(hydroxy-4 méthoxy-3 phényl)-2 hydroxy-2 éthyl] amino-3 butane répondant à la formule suivante :

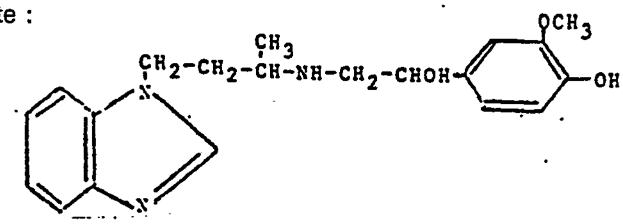

(I)

ainsi que ses sels d'addition d'acides minéraux ou organiques, non toxiques, pharmaceutiquement acceptables, et les hydrates desdits sels.

2. Sulfate basique de [benzimidazolyl-1]-1 N-[(hydroxy-4 méthoxy-3 phényl)-2 hydroxy-2 éthyl] amino-3 butane, et ses hydrates.

3. Dichlorhydrate de [benzimidazolyl-1]-1 N-[(hydroxy-4 méthoxy-3 phényl)-2 hydroxy-2 éthyl] amino-3 butane et ses hydrates.

4. Médicament utile notamment dans le traitement de l'asthme et dans les cas d'accouchement prématuré, caractérisé en ce qu'il est constitué par le [benzimidazolyl-1]-1 N-[(hydroxy-4 méthoxy-3 phényl)-2 hydroxy-2 éthyl] amino-3 butane ou un sel d'addition d'acide non toxique de ce dernier ou un hydrate dudit sel selon l'une des revendications 1 à 3.

5. Composition pharmaceutique caractérisée en ce qu'elle contient comme ingrédient actif un médicament selon la REVENDICATION

ASSOCIé au poids médicinal à un support ou véhicule non toxique, pharmaceutiquement acceptable.

6. Composition pharmaceutique selon la revendication 5, caractérisée en ce qu'elle est présentée sous une forme appropriée pour l'administration orale, telle que comprimé, gélule ou dragée, dosé à 0,5–10 mg de principe actif, ou sirop à une concentration de 0,1–2 mg/$cm^3$ de principe actif.

7. Composition pharmaceutique selon la revendication caractérisée en ce qu'elle est présentée sous une forme appropriée pour l'administration parentérale, telle que solution aqueuse à une concentration de 0,02 à 0,4 mg $cm_3$ de principe actif.

8. Composition pharmaceutique selon la revendication 5, caractérisée en ce qu'elle est présentée sous forme de suppositoire contenant 0,25 à 5 mg de principe actif par unité.

9. Procédé de préparation du [benzimidazolyl-1]-1, N-[(hydroxy-4 méthoxy-3 phényl)-2 hydroxy-2 éthyl] amino-3 butane répondant à la formule suivante :

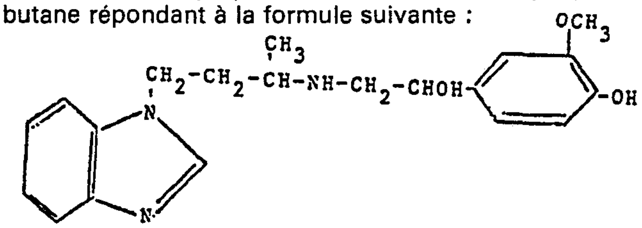

(I)

de ses sels d'addition d'ac ides minéraux ou organiques, non toxiques, pharmaceutiquement acceptables, et des hydrates desdits sels,

caractérisé en ce qu'il comporte les étapes suivantes :

a) condensation de l'amino-3 [benzimidazolyl-1]-1 butande avec le (méthoxy-3 phényl méthoxy-4) phénylglyoxal ou avec l'éthoxy-2 hydroxy-2 (méthoxy-3 phénylméthoxy-4 phényl)-1 éthanone, en milieu alcoolique, pour former une imine,

b) réduction in "situ" de cette imine par du borohydrure de sodium en excès pour former le [benzimidazolyl-1]-1 N-[hydroxy-2 (méthoxy-3 phénylméthoxy-4 phényl)-2 éthyl] amino-3 butane, suivie de la transformation de ce dernier en un sel d'addition d'acide non toxique, sous forme cristallisée,

c) hydrogénolyse dudit sel pour obtenir le sel correspondant du composé (I), que l'on récupère et que l'on purifie éventuellement,

d) éventuellement transformation du sel obtenu en la base correspondante désirée de formule I par alcalinisation en milieu aqueux au moyen d'un carbonate alcalin ou d'une base faible,

e) éventuellement salification de la base de formule I par un acide non toxique pour obtenir le sel correspondant désiré.

10. Procédé selon la revendication 9, caractérisé en ce que l'étape d'hydrogénolyse est conduite en solution ou en suspension hydroalcoolique, en présence de charbon palladié et d'hydrogène sous une pression de 1 à 50 bars à une température de 20 à 40°C.

11. Procédé selon la revendication 9, caractérisé en ce que la transformation en sel d'addition d'acide dans l'étape b) est effectuée par addition d'acide sulfurique en quantité nécessaire pour la salification de la moitié des fonctions basiques du [benzimidazolyl-1]-1, N-[(méthoxy-3 phénylméthoxy-4 phényl)-2 hydroxy-2 éthyl] amino-3 butane formé, en vue d'obtenir un sulfate basique.

12. Procédé selon la revendication 9, caractérisé en ce que la transformation en sel d'addition d'acide dans l'étape b) est effectuée par addition d'acide chlorhydrique.

13. Procédé selon la revendication 9, caractérisé en ce que la transformation en sel d'addit ion d'acide dans létape b) est effectuée par addition d'acide sulfurique en quantité nécessaire pour la salification de la totalité des fonctions basiques du [benzimidazolyl-1]-1, N-[(méthoxy-3 phénylméthoxy-4 phényl)-2 hydroxy-2 éthyl] amino-3 butane formé, en vue d'obtenir un sulfate neutre.

14. Procédé selon la revendication 9, caractérisé en ce que la transformation du sel en base correspondante dans l'étape b) est effectuée par -addition d'une solution saturée d'un carbonate alcalin -extraction de la base précipitée par un solvant tel que le dichlorométhane -séchage de l'extrait et évaporation du solvant d'extraction sous vide.

15. Procédé selon la revendication 9, caractérisé en ce que l'amino-3 [benzimidazolyl-1]-1 butane de départ est préparé par-condensation du benzimidazole avec la méthylvinylcétone en léger excès, en présence d'une quantité catalytique d'hydroxyde de benzyltriméthylammonium, au sein d'un solvant neutre apolaire, tel qu'un hydrocarbure aromatique ou l'acétate d'éthyle, pour former le [benzimidazolyl-1]-1 oxo-3 butane, -transformation de ce dernier en son oxime par de l'acétate d'hydroxylamine, -et hydrogénation catalytique de l'oxime, en milieu alcoolique en présence d'ammoniaque et de nickel de Raney sous une pression de 20 à 80 bars, et à une température de 50 à 100°C pour former le composé de départ désiré.

16. Procédé selon la revendication 9, caractérisé en ce que le (méthoxy-3 phényl méthoxy-4) phénylglyoxal de départ est préparé -par oxydation de la (méthoxy-3 phénylméthoxy-4 phényl)-1 éthanone au moyen de l'oxyde de sélénium, à 100°C, dans un solvant convenable, tel que le dioxanne.

17. Procédé selon la revendication 9, caractérisé en ce que le (méthoxy-3 phényl méthoxy-4) phénylglyoxal de départ est préparé par traitement de la (méthoxy-3 phényl méthoxy-4 phényl)-1 éthanone par le brome en

présence d'un oxyde d'alkyle inférieur, ou par le pyrrolidone-2 hydrotribromure dans un hydrocarbure gras chloré pour former la bromocétone correspondante, suivi du traitement de celle-ci par la N-N-diéthylhydroxylamine.

18. Procédé selon la revendication 9, caractérisé en ce que l'éthoxy-2 hydroxy-2 (méthoxy-3 phényl méthoxy-4 phényl)-1 éthanone est préparé par traitment du (méthoxy-3 phényl méthoxy-4) phénylglyoxal par l'éthanol.